# FASCICULE DE BREVET EUROPEEN

(11) **EP 0 365 427 B1**
(45) Date de publication et mention de la délivrance du brevet: **06.04.1994**
(21) Numéro de dépôt: 89402883.6
(22) Date de dépôt: 18.10.1989
(51) Int. Cl.: A61F 13/12, A61K 7/00

(54) **Nouvelles compresses palpebrales, leur procédé d'imbibition et les produits ainsi réalisés**
Augenlidkompressen, Verfahren zum Durchtränken derselben sowie durchtränkte Produkte
Eyelid compresses, process for soaking same and resultant products

(30) Priorité: 18.10.1988 FR 8813679
(43) Date de publication de la demande: 25.04.1990
(73) Titulaire: LABORATOIRE CHAUVIN SA, 34009 Montpellier Cédex 1 (FR)
(72) Inventeur: Bonjour, Patrick c/o Laboratoires Chauvin, F-34090 Montpellier (FR)
(74) Mandataire: Burtin, Jean-François (FR)

(56) Documents cités:
- DE-A- 2 836 707
- DE-A- 3 524 392
- FR-A- 2 606 612
- GB-A- 136 883
- US-A- 3 619 280

## Description

L'invention a pour objet un nouveau type de compresse palpébrale et son mode de réalisation.

Elle a plus particulièrement pour objet, un nouveau type de compresse anatomique destiné à l'application sur le pourtour des paupières.

Elle a spécifiquement pour objet une compresse palpébrale anatomique bi-oculaire présentante une forme oblongue quadratique à bords sensiblement arrondis possédant un côté longitudinal sensiblement rectiligne présentant des angles arrondis aux extrémités , un second côté longitudinal incurvé dont les deux sections sont réunies par un angle obtus, calculé de façon à épouser la forme de la base du nez, ces deux côtés longitudinaux étant réunis entre eux par des sections droites présentant à l'intersection des deux côtés longitudinaux une portion arrondie.

Des telles compresses sont décrites, par exemple, dans la publication de l'art anterient : DE-A-3 524 392.

La compresse anatomique selon l'invention est caractérisée comme specifié dans la deuxième partie de la revendication 1.

Les compresses palpebrales selon l'invention possèdent une longueur d'environ 10 à 18 cm, de préférence 15 à 17 cm et une hauteur variant entre 7 et 10 cm et de préférence 8 cm aux extrémités et de 5 à 8 cm à l'échancrure médiane, de préférence 6 cm. L'épaisseur moyenne est de l'ordre de 1 mm, elle peut être un peu supérieure ou un peu inférieure à cette valeur.

L'invention concerne également un procédé d'imbibition des compresses palpebrales définies ci-dessus qui consiste en ce que l'on imprègne par trempage, par immersion ou pulvérisation ledit matériau hydrophile, d'une solution aqueuse contenant un agent d'humidification, au moins un principe actif d'origine végétale et éventuellement un extrait aromatique obtenu par distillation de fleurs.

La solution d'imprégnation peut contenir en outre un agent tampon adapté au pH de la peau ainsi que des agents de conservation, des agents fluidifiants volatils, des agents colorants et/ou des agents odorants. L'agent d'humidification joue un rôle important car il permet de retenir l'eau appliquée sur la compresse palpebrale et d'éviter son évaporation pendant le temps le plus long possible. Ainsi, la compresse palpebrale demeure humide au moment de son application et aussi pendant tout le temps de son application et confère une sensation de fraicheur et de bien-être, lorsque les paupières sont irritées ou fatiguées.

Dans un mode d'exécution particulier, l'agent d'humidification est une silicone, un hibitane ou une lanoline hydrogénée et ou un alcool polyfonctionnel, non irritant, peu volatil et très soluble dans l'eau. Un tel agent est en particulier un glycol comme un polymère d'éthylène glycol, le propylène glycol, le dipropylène glycol ou le glycérol. La préférence va au propylène glycol qui permet une bonne imprégnation des compresses, qui retient bien l'humidification des compresses palpébrales et qui, bon solvant, contribue à lamise en solution du principe actif d'origine végétale et de l'extrait aromatique extrait de fleurs. La concentration en agent d'humidification peut varier dans de larges proportions et notamment de 0,5 à 5 % de la quantité d'eau mise en jeu. De préférence, la quantité d'agent d'humidification s'échelonne de 0,5 à 2 %.

Le principe actif d'origine végétale se définit par une très grande diversité d'origine selon l'usage bénéfique pour la santé auquel les compresses sont destinées. On pourra citer, à cet égard, les extraits aqueux, hydroglycoliques, hydroglycériques ou hydroalcooliques de plantes comme : l'Hamamelis, le Plantain, le Bleuet, la Mauve, la Camomille, le Pavot, la Violette, la Pensée sauvage, le Tussilage. La concentration en principe actif varie de 0,5 à 10 % selon l'usage auquel il est destiné et de préférence de 1 à 2 %. On peut utiliser une solution renfermant un principe acitf d'origine végétale ou plusieurs principes actifs d'origine végétale ou plusieurs principes actifs différents d'origine végétale.

L'extrait aromatique obtenu à partir de fleurs est de préférence une eau distillée obtenue par distillation ou par entrainement à la vapeur de principes aromatiques comme par exemple l'eau de rose, l'eau de lavande, l'eau de géranium, l'eau de menthe ou l'eau de Mélisse.

Selon un mode d'exécution actuellement préféré le principe actif d'origine végétale sont l'eau d'Hamamelis et/ou l'eau de Plantain et l'extrait aromatique est l'eau de Rose.

Le pH de la solution qui inhibe la compresse palpébrale est au voisinage de celui du pH des sécretions lacrymales et est compris entre 7 et 7,6.

L'agent de conservation introduit dans la solution d'imbibition des compresses palpébrales peut être un ester de l'acide p.Hydroxy benzoïque comme le paraben méthylique ou éthylique, un sel d'ammonium quaternaire comme un sel de Benzalkonium ou un sel de Cetrimonium.

L'invention s'étend également au procédé de conditionnement des compresses palpébrales imbibées selon l'invention caractérisée en ce que les compresses imbibées sont pliées d'une manière asymétrique de façon à laisser une partie plus courte et une partie plus longue permettant une préhension plus aisée lors du dépliage et sont réparties en conditionnement unitaire sous sachet étanche.

De préférence, le conditionnement unitaire est un sachet en polyéthylène revêtu d'une pellicule d'aluminium.

Les compresses palpébrales ainsi réalisées et conditionnnées en sachets unitaires gardent ainsi toute leur humidité et les principes actifs dans leur intégralité. Leur application entraine une sensation de fraicheur ainsi qu'un soulagement des phénomènes d'irritation ou des phénomènes de gonflement des paupières liés à un environnement irritant ou à des conditions de travail peu favorable ou bien encore, à la survenue d'un corps étranger dans l'oeil ou sur l'oeil comme des poussières ou bien encore une particule métallique.

Les exemples suivants illustrent l'invention sans toutefois la limiter.

### EXEMPLE I

Compresses palpébrales à l'eau d'Hamamelis et à l'eau distillée de Rose.

On réalise par addition successive et homogénéisation une solution d'imbibition constituée par :

| | |
|---|---|
| - eau distillée d'Hamamélis | 10 ml |
| - eau distillée de Rose centifolia | 10 ml |
| - Propylène glycol | 1 g |
| - chlorure du benzalkonium | O,O1 g |
| - eau | q.s.p 100 ml |

On trempe dix compresses palpébrales dans cette solution pendant 5 minutes. On les laisse ensuite s'égoutter puis on les presse très légèrement pour retirer le peu de liquide qui pourrait encore exsuder à la surface. On les plie ensuite en quatre volets d'une manière asymétrique de façon à avoir un grand pan inférieur et un pan plus petit extérieur. On les répartit unitairement dans des complexes formés d'une feuille de polythène et d'une feuille d'aluminium que l'on soude pour former un conditionnement unitaire étanche.

Les compresses palpébrales en application matin et soir sur les paupières préalablement démaquillées si c'est nécessaire, constituent un traitement tonique et relaxant des paupières fatiguées en hydratant et réhydratant les tissus.

### EXEMPLE II

### Compresses à l'extrait de plantain

On réalise le mélange suivant :

| | |
|---|---|
| - eau distillée d'Hamamélis | 10 ml |
| - extrait aqueux de Plantain | 1 ml |
| - chlorure de benzalkonium | 0,01 g |
| - système tampon q.s.p pour pH | 7,2 |
| - propylène glycol | 2 g |
| - eau purifiée q.s.p | 100 ml |

Cette solution sert à imprégner vingt compresses palpébrales qui sont pliées et conditionnées selon le mode opératoire de l'exemple I.

Les compresses à l'extrait de plantain contribuent à l'amélioration de l'oeil rouge, des yeux qui tirent, des yeux secs après séjour dans un environnement d'air sec (air conditionné chaud ou froid, chauffage central par le plancher..) séjours au soleil ou à la montagne.

## Revendications

1. Une compresse palpébrale anatomique bioculaire présentant une forme oblongue quadratique, dont l'extrémité de chaque côté présente des angles arrondis et dont le second côté longitudinal présente une légère incurvation en son centre pour reposer sur la base du nez, les deux côtés longitudinaux étant réunis entre eux par deux sections droites présentant à l'intersection des deux côtés longitudinaux, une portion arrondie, caractérisée en ce que ladite compresse palpébrale est formée d'un matériau hydrophile mince, souple et absorbant choisi parmi les cotons à longues fibres décatis et dégraissés d'un poids spécifique compris entre 100 et 150 g/m2, dont le numéro métrique est de 50 dans le sens de la chaîne et de 14 dans le sens de la trame au niveau du fil et dont le numéro métrique est de 12 à 20 dans le sens de chaîne et de 10 à 15 dans le sens de la trame au niveau de la trame, dont l'épaisseur est de l'ordre de 1mm et dont la capacité d'absorption est voisine de 10 g de soluté aqueux par compresse.

2. Une compresse palpébrale anatomique bioculaire selon la revendication 1° caractérisée en cequ'elle est imprégnée d'une solution aqueuse hydro-alcoolique, hydro-glycolique ou hydro-glycérique d'un extrait de plante choisie dans le groupe constitué par l'Hamamélis, le Plantain, le Bleuet, la Mauve, la Camomille, le Pavot, la Violette, la Pensée sauvage et le Tussilage.

## Claims

1. A biocular anatomic dressing for the eye lids showing an oblong quadratic shape, an end of which, on each side, displays rounded angles and the second longitudinal length shows a small incurvation in its middle in order to rest on the bottom of the nose, the two longitudinal lengths being united between them by two right sections showing at the intersection of these two longitudinal sides a rounded part characterized in that the said dressing for eye lids is made of a thick hydrophilic material which is supple and absorbing, selected from the long fibers, steamed and scoured cotton, the specific weight of which being comprised between 100 and 150 g/m2, the metric number of which being 50 in the way of the warp end and being 14 in the way of the weft, at the level of the thread, and the metric number tehreof is 12 to 20 and 10 to 15 for the weft yarn, at the level of the weft, the thickness of which is of the order of 1 mm and the capacity of absorption of which is near from 10 g aqueous solute per dressing.

2. A biocular anatomic dressing for the eye lids according to claim 1° characterized in that it is soaked with an aqueous hydroalcoholic, hydroglycolic or hydroglyceric solution of a plant extract selected from the group consisting of Hamamelis , Plantain, Bluet, Mallow, Camomile, Poppy, Violet, Wild Pansy and Coltsfoot.

## Patentansprüche

1. Eine binokulare anatomische Kompresse fur Augenlid die eine quadratisches längliches Gestalt aufweist, deren Ende auf jede Seite rundliche Winkel vorliegen und deren zweite Längsseite eine leise Verkrummung in seiner Mitte um auf dem Nase zu liegen vorliegt, deren beide Längsseite untereinander mit zwei gerade Abschnitte die am Schnittfläche der beide Längsseite eine rundliche Teil vorliegen, vereinigen sind, dadurch gekennzeichnet ist dass diese Kompresse fur Augenlid, aus einer Wasser aufsaugende, feine, weiche und aufnehmende Material besteht die aus der Gruppe der gekrimpte und entfettene, mit Länge Fasern, Baumwolle, dessen spezifisches Gewicht zwischen 100 und 150 g/m2 enthalten ist, dessen metrischen Zahl 50 in der Richtung des Fliessbands und 14 in der Richtung des Schusses am Stand der Faden und dessen metrischen Zahl von 12 bis 20 in der Richtung des Fliessbands und von 10 bis 15 in der Richtung des Schusses am Stand des Schusses, dessen Dicke in der Ordnung von 1 mm ist, und dessen Aufnahmefähigkeit in der Nahe von 10 g wasserige Lösung per Kompresse liegt.

2. Eine binokulare anatomische Kompresse für Augenlid, nach Anspruch 1°, dadurch gekennzeichnet ist, dass sie mit einer wasserige, hydroalkoholische, hydroglykolische, oder hydroglyzerinische Lösung einer planzischen Extrakt durchgetrankt ist, der aus der Gruppe von Hamamelis, Wegerich, Kornblume, Malve, Kamille, Mohn, Veilchen, Stiefmüterchen und Huftlattisch ausgewählt ist.
